# EUROPÄISCHE PATENTSCHRIFT

(11) **EP 1 565 222 B1**
(45) Veröffentlichungstag und Bekanntmachung des Hinweises auf die Patenterteilung: **30.04.2008**
(21) Anmeldenummer: 03767571.7
(22) Anmeldetag: 19.11.2003
(51) Int. Cl.: A61M 5/28, A61M 5/315

(54) **VORGEFÜLLTE SPRITZE**
PREFILLED SYRINGE
SERINGUE PREREMPLIE

(30) Priorität: 21.11.2002 DE 10254321
(43) Veröffentlichungstag der Anmeldung: 24.08.2005
(73) Patentinhaber: Arzneimittel GmbH Apotheker Vetter & Co. Ravensburg, 88212 Ravensburg (DE)
(72) Erfinder: VETTER, Udo, J., 88214 Ravensburg (DE); GLOCKER, Joachim, 88250 Weingarten (DE); ALBERSTETTER, Jochen, 88212 Ravensburg (DE)
(74) Vertreter: Gleiss, Alf-Olav
(86) Internationale Anmeldenummer: PCT/EP2003/012924
(87) Internationale Veröffentlichungsnummer: WO 2004/045681

(56) Entgegenhaltungen:
- WO-A-00/16829
- DE-A- 19 638 940
- US-A- 5 456 388
- US-B1- 6 331 173

## Beschreibung

Die Erfindung betrifft eine vorgefüllte Spritze für medizinische Zwecke mit einem Spritzenzylinder, einem darin angeordneten, mittels einer Kolbenstange verschiebbaren Spritzenkolben, einem das kanülenseitige Ende des Spritzenzylinders verschließenden Verschlussstopfen, der einen mittels einer Membran verschließbaren Durchgangskanal aufweist, einer an dem dem Verschlussstopfen gegenüberliegenden Ende des Spritzenzylinders angeordneten, eine Durchgangsöffnung für die Kolbenstange aufweisenden Fingerauflage und mit einer Gewindeeinrichtung, die mit der Kolbenstange und mit der Fingerauflage zusammenwirkt.

Spritzen der hier angesprochenen Art sind bekannt (DE 196 38 940 C2). Sie zeichnen sich dadurch aus, dass der Innenraum des Spritzenzylinders durch die Membran im Verschlussstopfen dicht abgeschlossen ist. Wenn der Inhalt der Spritze ausgetragen werden soll, muss die Membran gesprengt werden. Dies erfolgt dadurch, dass durch Verlagerung des Spritzenkolbens ein Überdruck im Spritzenzylinder aufgebaut wird, so dass die Membran schließlich platzt. Es ist auch bekannt, einen Dorn vorzusehen, der so angeordnet ist, dass er bei einer Auslenkung der Membran diese zerstört und der Inhalt der Spritze über eine Kanüle austreten kann. Es ist darüber hinaus bekannt, eine Gewindeeinrichtung vorzusehen, die einen definierten Vorschub der Kolbenstange und des damit verbundenen Spritzenkolbens ermöglicht. Dazu ist die Kolbenstange mit einem Außengewinde versehen, das mit einem im Bereich der Fingerauflage vorgesehenen Innengewinde zusammenwirkt.

Es hat sich herausgestellt, dass der Zeitpunkt der Zerstörung der Membran nicht immer genau reproduzierbar ist. Manchmal platzt die Membran noch nicht, wenn die Kolbenstange über den vollständigen Gewindeweg verlagert ist. Manchmal platzt die Membran so früh, dass das Gewinde der Kolbenstange noch in Eingriff steht mit dem Gewinde in der Fingerauflage. Es ist dann erforderlich, die Kolbenstange weiterzudrehen, bis das Außengewinde der Kolbenstange ganz durch das Innengewinde der Fingerauflage hindurchgeschraubt ist. Erst dann kommt die Kolbenstange von der Fingerauflage frei und ist axial verschiebbar ist. Dann erst kann die Kolbenstange den Spritzenkolben verlagern und den Inhalt des Spritzenzylinders austragen.

Aufgabe der Erfindung ist es daher, eine vorgefüllte Spritze der eingangs genannten Art zu schaffen, die diesen Nachteil nicht aufweist.

Zur Lösung dieser Aufgabe wird eine vorgefüllte Spritze vorgeschlagen, die die in Anspruch 1 genannten Merkmale aufweist. Sie zeichnet sich dadurch aus, dass die Gewindeeinrichtung eine mit der Fingerauflage lösbar verbundene Gewindehülse mit einem Innengewinde aufweist, das mit einem Außengewinde an der Kolbenstange zusammenwirkt. Die Gewindeeinrichtung erlaubt es, die Kolbenstange so zu verdrehen, dass diese in das Innere des Spritzenzylinders hineinverlagert wird, wobei auch der Spritzenkolben in Richtung auf das kanülenseitige Ende des Spritzenzylinders verschoben wird. Dies führt zu einem Überdruck im Inneren des Spritzenzylinders und zu einer Auswölbung der Membran. Sobald diese aufreißt, kann die Drehbewegung der Kolbenstange eingestellt werden. Diese kann nun vielmehr mit einer in Richtung der Kolbenstange wirkenden Kraft beaufschlagt werden, um den Spritzenkolben im Inneren des Spritzenzylinders zu verlagern. Sollte das Außengewinde der Kolbenstange noch mit dem Innengewinde der Gewindehülse in Eingriff stehen, kann diese durch die in Richtung der Kolbenstange wirkende Kraft aus der Fingerauflage herausgedrückt werden. Dadurch, dass die Fingerauflage also nicht starr mit der Kolbenstange gekoppelt ist, kann die axiale Vorschubbewegung der Kolbenstange und des Spritzenkolbens auch dann erfolgen, wenn die Membran bereits zu einem Zeitpunkt aufreißt, zu dem das Außengewinde der Kolbenstange noch mit dem Innengewinde der Gewindehülse in Eingriff steht.

Weitere Vorteile ergeben sich aus den Unteransprüchen.

Die Erfindung wird im Folgenden anhand der Zeichnung näher erläutert. Es zeigen:
- Figur 1: eine Spritze im Längsschnitt in einer ersten Funktionsstellung;
- Figur 2: die in Figur 1 wiedergegebene Spritze in einer zweiten Funktionsstellung;
- Figur 3: die in den Figuren 1 und 2 dargestellte Spritze in einer dritten Funktionsstellung;
- Figur 4: eine vergrößerte Darstellung einer Fingerauflage im Längsschnitt;
- Figur 5: eine Explosionsdarstellung der in Figur 4 dargestellten Fingerauflage und
- Figur 6: eine vergrößerte Darstellung des verschlossenen kanülenseitigen Endes einer Spritze im Längsschnitt.

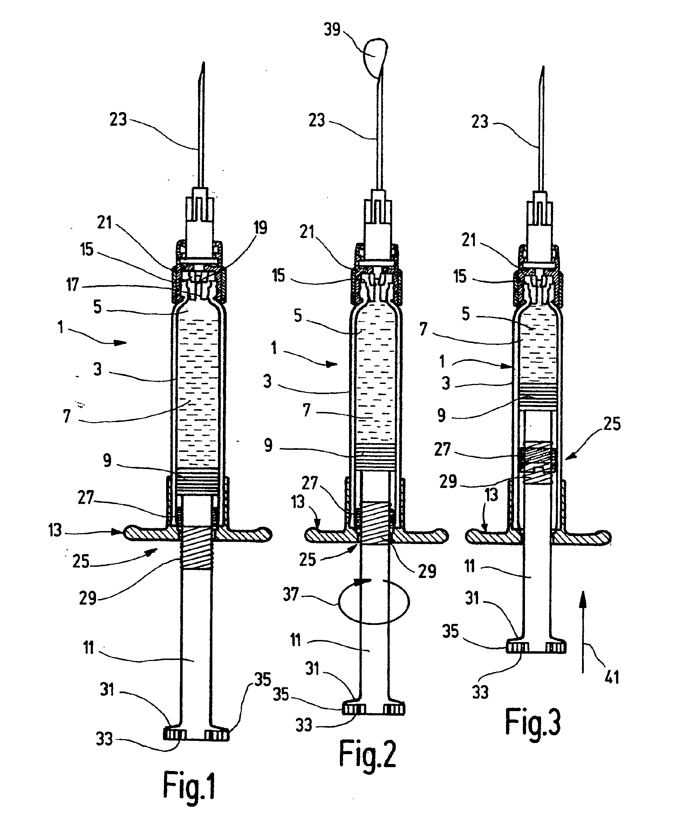

Die in Figur 1 dargestellte Spritze 1 weist einen hohlen Spritzenzylinder 3 auf, dessen Innenraum 5 mit einer für eine Injektion bereitgestellten Substanz 7 gefüllt ist. Im Innenraum 5 ist ein Spritzenkolben 9 in Längsrichtung des Spritzenzylinders 3 beweglich gelagert. Auf bekannte Weise ist der Außendurchmesser des elastischen Spritzenkolbens etwas größer gewählt als der Innendurchmesser des Spritzenzylinders 3, so dass der Innenraum 5 einerseits durch den Spritzenkolben 9 dicht abgeschlossen wird, andererseits bei einer Verlagerung des Spritzenkolbens 9 die Substanz 7 nicht im Bereich des Spritzenkolbens 9 aus dem Innenraum 5 austreten kann, so dass ein Überdruck aufgebaut wird.

Der Spritzenkolben 9 ist mit einer Kolbenstange 11 gekoppelt. Diese durchgreift eine Fingerauflage 13, die mit dem Spritzenzylinder 3 fest gekoppelt ist, beispielsweise durch eine Rastverbindung. An dem der Fingerauflage 13 gegenüberliegenden kanülenseitigen Ende des Spritzenzylinders 3 ist ein Verschlussstopfen 15 mit einem Durchgangskanal 17 vorgesehen, der durch eine Membran 19 verschlossen wird.

Der Verschlussstopfen 15 ist auf bekannte Weise in das sich verjüngende Ende des Spritzenzylinders 3 eingesteckt und wird von einer Aufprellkappe 21 gehalten, in die eine Kanüle 23 eingebracht ist.

An dem der Kanüle 23 gegenüberliegenden Ende der Spritze 1 ist eine Gewindeeinrichtung 25 vorgesehen, die die Kolbenstange 11 von der Fingerauflage 13 entkoppelt. Die Gewindeeinrichtung 25 umfasst eine Gewindehülse 27, die mit der Fingerauflage 13 lösbar verbunden und mit einem Innengewinde versehen ist, das in ein auf der Außenseite der Kolbenstange 11 vorgesehenes Außengewinde 29 eingreift.

Das der Kanüle 23 abgewandte, freistehende Ende 31 der Kolbenstange 11 kann mit einer Druckplatte 33 versehen sein, die an ihrer Umfangsfläche mit einer Riffelung 35 versehen ist, um die Griffigkeit der Kolbenstange 11 zu erhöhen. Dies erleichtert ein Verdrehen der Kolbenstange 11 gegenüber dem Spritzenzylinder.

Die in Figur 1 dargestellte Spritze 1 befindet sich in einer ersten Funktionsstellung, in der der Spritzenkolben 9 in seiner Ausgangslage angeordnet ist, nämlich in einem maximalen Abstand zum Verschlussstopfen 15.

In dieser Funktionsstellung ist der Innenraum 5 in der Regel nicht mit einem Überdruck beaufschlagt, so dass die Membran 19 unbelastet und nicht ausgewölbt ist. Über der Substanz 7 kann sich noch ein gas- beziehungsweise luftgefüllter Freiraum im Innenraum 5 des Spritzenzylinders 3 befinden.

In dieser Funktionsstellung greift das Außengewinde 29 der Kolbenstange 11 vorzugsweise noch nicht in das Innengewinde der Gewindehülse 27 ein. Es ist möglich, um diesen Eingriff herbeizuführen, die Kolbenstange 11 in axialer Richtung der Spritze 1 etwas in Richtung auf die Kanüle 23 zu verschieben. Dadurch wird das in dem Freiraum oberhalb der Substanz 7 im Innenraum 5 vorhandene Gas etwas komprimiert. Diese Ausgestaltung hat den Vorteil, dass bei einer unbeabsichtigten Verdrehung der Kolbenstange 11 diese nicht ohne weiteres in axialer Richtung des Spritzenzylinders 3 verlagert wird.

Denkbar ist es aber auch, dass bereits in der Funktionsstellung gemäß Figur 1 sich das Außengewinde 29 in Eingriff befindet mit dem Innengewinde der Gewindehülse 27. Eine versehentliche Verlagerung der Kolbenstange 11 kann in diesem Fall auf andere Weise verhindert werden.

Schließlich ist es noch möglich, in der Fingerauflage selbst ein kurzes Gewinde vorzusehen, das einerseits dazu dient, beim Zusammenbau der Spritze 1 die Kolbenstange 11 in die Fingerauflage 13 einzuführen, und andererseits dazu, als Schutz gegen eine versehentliche axiale Verlagerung der Kolbenstange 11 gegenüber der Fingerauflage 13 zu dienen. Diese einfache Möglichkeit, eine unbeabsichtigte axiale Verlagerung der Kolbenstange 11 zu vermeiden, trägt zu einem wesentlichen Sicherheitsfaktor bei.

Figur 2 zeigt die Spritze gemäß Figur 1 in einer zweiten Funktionsstellung. Gleiche Teile sind mit gleichen Bezugsziffern versehen, so dass auf die Beschreibung zu Figur 1 verwiesen wird, um Wiederholungen zu vermeiden.

Die zweite Funktionsstellung gemäß Figur 2 zeichnet sich dadurch aus, dass entsprechend dem Pfeil 37 eine Verdrehung der Kolbenstange 11 durchgeführt wurde, wobei das Außengewinde 29 der Kolbenstange 11 mit dem Innengewinde der Gewindehülse 27 kämmt und bei entsprechender Drehrichtung der Kolbenstange 11 diese in das Innere des Spritzenzylinders 3 hineinverlagert wird. Dadurch wird der mit der Kolbenstange 11 gekoppelte Spritzenkolben 9 in Richtung auf die Kanüle 23 verschoben. Dies führt zu einem Überdruck im Innenraum 5. Dadurch wölbt sich die Membran 19 nach außen, das heißt, in Richtung auf die Kanüle 23. Sie wird schließlich durch den Innendruck zerrissen. Das Platzen der Membran ist hör-und fühlbar, weil die zum Drehen der Kolbenstange 11 erforderliche Kraft merklich kleiner wird. Bei einer weiteren Drehung der Kolbenstange 11 bewegt sich der Spritzenkolben 9 weiter in Richtung auf die Kanüle 23, so dass im Innenraum 5 vorhandenes Gas ausgetrieben und die Spritze 1 definiert entlüftet wird. Schließlich kann ein Tropfen 39 der Substanz 7 aus der Kanüle 23 austreten, so dass ein Benutzer erkennen kann, dass die Membran 19 geplatzt und die Spritze 1 entlüftet ist. Es zeigt sich also, dass die Gewindeeinrichtung 25 auf einfache Weise ein exaktes und feinfühliges Entlüften der Spritze 1 ermöglicht.

Figur 2 zeigt, dass bei dem hier dargestellten Ausführungsbeispiel der Spritze 1 in der zweiten Funktionsstellung das Außengewinde der Kolbenstange 11 noch in Eingriff steht mit dem Innengewinde der Gewindehülse 27. Es ist hier also noch eine axiale Kopplung zwischen der Kolbenstange 11 und der Fingerauflage 13 gegeben. Da die Fingerauflage 13 fest mit dem Spritzenzylinder 3 verbunden ist, kann bei einer derartigen Kopplung grundsätzlich die Kolbenstange 11 nicht weiter in Richtung auf die Kanüle 23 verlagert werden. Hier ist jedoch vorgesehen, dass die Gewindeeinrichtung 25 eine Gewindehülse 27 aufweist, die lösbar mit der Fingerauflage 13 verbunden ist. Durch einen in Richtung auf die Kanüle 23 wirkenden Druck, der auf die Kolbenstange 11 ausgeübt wird, kann die Gewindehülse 27 von der Fingerauflage 13 getrennt und damit die Kolbenstange 11 gemeinsam mit dem Zylinderkolben 9 in den Innenraum 5 des Spritzenzylinders 3 hineingeschoben werden, um die Substanz 7 durch die Kanüle 23 auszutragen.

Figur 3 zeigt die Spritze 1 in ihrer dritten Funktionsstellung, in der durch eine Kraft, die durch den Pfeil 41 angedeutet ist, die Kolbenstange 11 gemeinsam mit dem Spritzenkolben 9 in Richtung auf die Kanüle 23 verlagert ist. Deutlich erkennbar wird hier, dass die Gewindehülse 27 von der Fingerauflage 13 getrennt und gemeinsam mit der Kolbenstange 11 in den Innenraum 5 verlagert wurde.

Teile, die bereits anhand der Figuren 1 und 2 erläutert wurden, sind in Figur 3 mit gleichen Bezugsziffern versehen, so dass insofern auf eine Wiederholung der Beschreibung verzichtet wird.

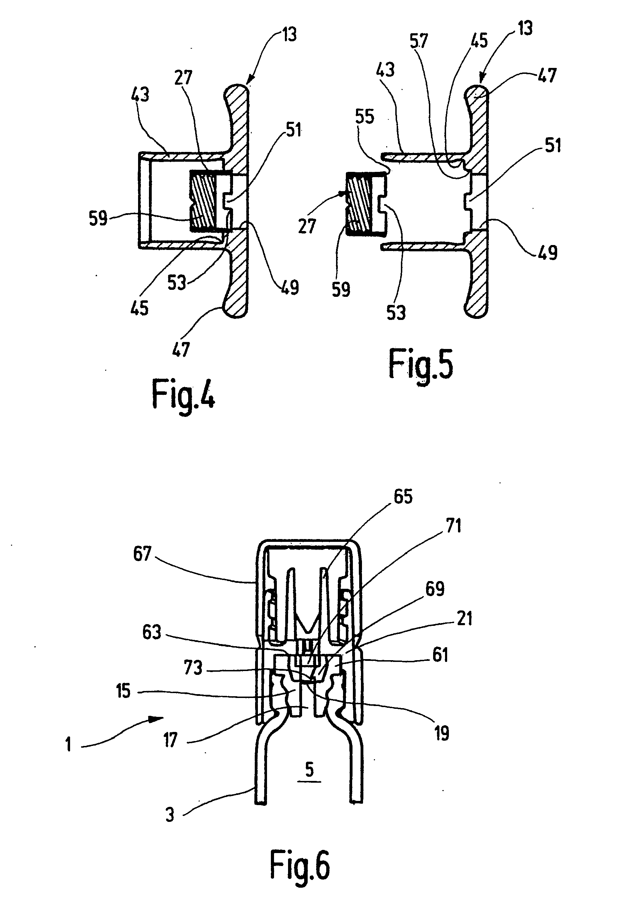

Figur 4 zeigt in vergrößerter Darstellung die Fingerauflage 13 gemeinsam mit der Gewindehülse 27. Die beiden Teile sind im Längsschnitt dargestellt.

Die Darstellung lässt erkennen, dass die Fingerauflage mit einem zylindrischen Ansatz 42 versehen sein kann, der das der Kanüle 23 abgewandte Ende des Spritzenzylinders 3 umgreift und mit einer innen liegenden Ringnut 45 versehen ist, in die ein umlaufender Ringwulst am Ende des Spritzenzylinders 3 eingreifen kann. Dadurch wird die Fingerauflage 13 sicher am Spritzenzylinder 3 gehalten.

Die Fingerauflage weist eine Fingerplatte 47 mit einer Durchgangsöffnung 49 auf, die in der Regel konzentrisch zur Mittelachse des Spritzenzylinders 3 angeordnet ist und von der hier nicht dargestellten Kolbenstange 11 durchgriffen wird. Im Inneren des zylindrischen Ansatzes 43 liegt die Gewindehülse 27 der Gewindeeinrichtung 25. Sie ist als separates Teil ausgebildet und lösbar mit der Fingerauflage 13 verbunden.

Es ist grundsätzlich möglich, die Gewindehülse 27 in die Fingerplatte 47 einzupressen und damit zu verankern. Bei dem hier dargestellten Ausführungsbeispiel weist die Gewindehülse 27 jedoch einen Ringwulst auf, der in eine Ringnut eingreift, die in der Fingerplatte 47 vorgesehen ist. Damit ist ein verbesserter Halt der Gewindehülse 27 in der Fingerplatte 47 sichergestellt.

Um eine Verdrehsicherung der Gewindehülse 27 gegenüber der Fingerauflage 13 zu gewährleisten, kann es -wie gesagt- ausreichen, die Gewindehülse in den Grundkörper der Fingerauflage 13 einzupressen, insbesondere dann, wenn beispielsweise der Ringwulst der Gewindehülse 27 mit Riffeln versehen ist, die in die in die Fingerplatte 47 eingebrachte Ringnut eingreifen.

Vorzugsweise ist hier jedoch ein Formschluss vorgesehen, der durch einen von der Fingerplatte 47 entspringenden Vorsprung 51 realisiert wird, der in eine in die Gewindehülse 27 eingebrachte Nut 53 eingreift. Der Formschluss kann auch dadurch erreicht werden, dass - umgekehrt- an der Gewindehülse 27 ein Vorsprung vorgesehen ist, der in eine Nut in der Fingerplatte 47 eingreift. Überdies ist es möglich, mehrere Vorsprünge und Nuten vorzusehen, um eine Relativdrehung zwischen Gewindehülse 27 und Fingerauflage 13 sicher zu verhindern.

Figur 5 zeigt eine Explosionsdarstellung der in Figur 4 dargestellten Fingerplatte 13. Gleiche Teile sind mit gleichen Bezugsziffern versehen, so dass insofern auf die Beschreibung zu Figur 4 verwiesen wird. Aus der Explosionsdarstellung wird deutlich, dass die Gewindehülse 27 auf ihrer Außenseite mit einem Ringwulst 55 versehen ist, der in eine Ringnut 57 in der Fingerplatte 47 eingreift. Die Darstellung zeigt auch deutlich den als Positioniernocken dienenden Vorsprung 51 und die zugehörige als Nut 53 bezeichnete Ausnehmung in der Gewindehülse 27.

Die Darstellungen gemäß den Figuren 4 und 5 lassen erkennen, dass die Höhe des Innengewindes 59 so gewählt ist, dass dieses sich nicht über den gesamten Innenraum der Gewindehülse 27 erstreckt.

Die Höhe des Innengewindes 59 wird so gewählt, dass die Gewindeeinrichtung 25 eine ausreichende Kraft aufbringen kann, um den Spritzenkolben 9 im Innenraum 5 zu verlagern und um einen Innendruck aufzubauen, der zum Platzen der Membran 19 führt.

Figur 6 zeigt vergrößert das kanülenseitige Ende der Spritze 1. Gleiche Teile sind mit gleichen Bezugsziffern versehen, so dass auf die Beschreibung der vorangegangenen Figuren verwiesen wird.

Die Vergrößerung zeigt insbesondere den Verschlussstopfen 19, der in den Endbereich des Spritzenzylinders 3 eingesteckt ist und durch einen geeigneten Formschluss dort sicher gehalten wird. Er weist außerdem eine Ringschulter 61 auf, die auf der Stirnseite des Spritzenzylinders 3 aufliegt und von einer geeigneten Halteschulter 63 der Aufprellkappe 21 gehalten wird.

Auf das freie Ende 65 der Aufprellkappe 21 ist ein Tip Cap aufgesetzt, der in dieser Position durch einen Originalitätsverschluss 67 gehalten wird.

In das Innere des Verschlussstopfens 15 ist ein Einsatz 69 eingebracht, der vorzugsweise aus Kunststoff besteht und einen Filter 71 umfasst, durch den die im Inneren der Spritze 1 vorhandene Substanz 7 ausgetragen wird.

Aus Figur 6 wird deutlich, dass der Durchgangskanal 17 im Verschlussstopfen 15 durch die Membran 19 verschlossen wird und sich durch den Einsatz 69 hindurch erstreckt. In einem Abstand oberhalb der Membran ist hier ein vom Einsatz 69 in den Durchgangskanal 17 vorspringender Dorn 73 erkennbar, dessen Spitze in Richtung auf die Membran 19 weist. Er ist so angeordnet, dass er die Membran 19 aufritzt, wenn diese sich bei einem Überdruck im Innenraum 5 der Spritzenkanüle 3 nach oben, das heißt in Richtung der aufzusetzenden Kanüle, wölbt. Diese Ausgestaltung ermöglicht ein sehr viel genauer reproduzierbares Aufplatzen der Membran 19 bei einem Überdruck im Innenraum 5.

Im Folgenden wird auf die Funktion der Spritze 1 näher eingegangen:

Die vorgefüllte Spritze 1 enthält eine Substanz 7, die im Innenraum 5 des Spritzenzylinders 3 durch die Membran 19 dicht abgeschlossen ist. Nach der Fertigstellung der Spritze 1 befindet sich die Kolbenstange 11 in ihrer zurückgezogenen Position, die sich aus Figur 1 ergibt. Die Spritze 1 ist mit einer Gewindeeinrichtung 25 versehen, die es erlaubt, durch eine Drehung der Kolbenstange 11 diese in axialer Richtung zu verlagern und den Spritzenkolben 9 im Innenraum 5 in Richtung auf den Verschlussstopfen 15 zu verschieben.

Vorzugsweise ist die Gewindeeinrichtung 25 so ausgelegt, dass die Kolbenstange 11 in der ersten Funktionsstellung gemäß Figur 1 frei drehbar ist, ohne dass dabei das Außengewinde 29 der Kolbenstange 11 mit dem Innengewinde 59 der Gewindehülse 27 in der Fingerauflage 13 in Eingriff steht. Eine Aktivierung der Gewindeeinrichtung 25 erfolgt dadurch, dass die Kolbenstange 11 etwas in den Spritzenzylinder 3 hineingedrückt wird, so dass die beiden Gewindebereiche ineinander greifen.

Die Gewindeeinrichtung 25 bewirkt dann, wie in Figur 2 dargestellt, eine Verlagerung des Spritzenkolbens 9, was zu einem Überdruck im Innenraum 5 führt, der die Membran 19 in Richtung zur Kanüle 27 vorwölbt. Bei einer ersten -bevorzugten- Ausführungsform führt die Wölbung allein schließlich zum Bersten der Membran 19, wenn die Kolbenstange 11 weiter in das Innere des Spritzenzylinders 3 eingeschraubt wird. Es ist jedoch auch möglich, wie anhand von Figur 6 erläutert, in unmittelbarer Nähe der Membran 19 einen Dorn 73 vorzusehen, der die Membran 19 bei einer Vorwölbung einritzt und zum definierten Bersten führt.

Die Gewindeeinrichtung 25 ist so ausgelegt, dass die Drehbewegung der Kolbenstange 11 in der Fingerauflage 13 entkoppelt ist von einer axialen Bewegung der Kolbenstange 11 in den Innenraum 5 des Spritzenzylinders 3. Dies hat den Vorteil, dass nach dem Bersten der Membran 19 und gegebenenfalls nach dem Entlüften des Innenraums 5 die axiale Verlagerung der Kolbenstange unabhängig von der momentanen Position des Außengewindes 29 im Innengewinde 59 durchgeführt werden kann: wie in Figur 3 dargestellt, löst sich bei einem Druck auf die Kolbenstange 11 in Richtung des Pfeils 41 die Gewindehülse 27 aus der Fingerplatte 47 der Fingerauflage 13, so dass die Kolbenstange 11 gemeinsam mit dem Spritzenkolben 9 unabhängig von der Gewindeeinrichtung 25 frei beweglich ist. Diese Ausgestaltung stellt sicher, dass die axiale Entkopplung der Kolbenstange 11 von der Fingerauflage 13 nicht mehr von der Systemgenauigkeit und von Toleranzen abhängig ist, die bei der Herstellung der Spritze gegeben sind.

Die Funktion der Gewindeeinrichtung 25 wird noch dadurch sichergestellt, dass die Gewindehülse 27 in der Fingerplatte 47 sicheren Halt findet und dort verdrehsicher gehalten wird. Dies kann durch einen Presssitz geschehen oder aber durch einen Formschluss, der anhand der Figuren 4 und 5 näher erläutert wurde. Vorzugsweise ist vorgesehen, dass die Gewindehülse 27 durch einen Eingriff eines Vorsprungs 51, der von der Fingerplatte 47 ausgeht, in einer Nut 53 gehalten wird, die an der Gewindehülse 27 vorgesehen ist.

Die Gewindeeinrichtung 25 ist als separates Teil, also getrennt von der Fingerauflage 13 und von der Kolbenstange 11 ausgebildet und so ausgelegt, dass auch Spritzen 1 mit dieser nachgerüstet werden können, sofern eine Kolbenstange 11 mit einem Außengewinde 29 vorgesehen ist.

Nach allem wird deutlich, dass bei der hier erläuterten Spritze 1 die Fingerauflage 13 selbst kein Innengewinde aufweist, sondern vielmehr zweiteilig ausgebildet ist. Sie umfasst eine Gewindehülse 27 einer Gewindeeinrichtung 25, die als separates Teil lösbar mit der Fingerauflage 13 verbunden ist, so dass die Drehbewegung der Kolbenstange 11 von deren axialen Verlagerung vollständig getrennt ist: Auch wenn das Außengewinde der Kolbenstange 25 noch mit dem Innengewinde 59 der Gewindehülse 27 kämmt, ist die Kolbenstange 11 in axialer Richtung verlagerbar. Die Drehbewegung der Kolbenstange 11 erfolgt also nur so lange, bis die Membran 19 sicher aufgesprengt und der Innenraum 5 der Spritze 1 entlüftet ist.

Dadurch, dass die Gewindehülse beim axialen Einschieben der Kolbenstange 11 in den Innenraum 5 von der Fingerauflage 13 getrennt wird, wird eine zusätzliche Sicherheit im Gebrauch der Spritze 1 erreicht: Durch Zurückdrehen der Kolbenstange 11 kann die Gewindehülse 47 nicht mehr in ihre Ausgangsposition zurückgesetzt werden. Eine neue Verwendung der Spritze 1 ist damit nicht mehr möglich. Es wird also verhindert, dass eine verunreinigte Spritze wieder in den Verkehr gebracht wird, und sichergestellt, dass diese nur einmal verwendbar ist.

## Patentansprüche

1. Vorgefüllte Spritze für medizinische Zwecke, mit einem einen Innenraum (5) einschließenden Spritzerzylinder (3) einem darin angeordneten, mittels einer ein Außengewinde (29) aufweisenden Kolbenstange (11) verschiebbaren Spritzenkolben (9), einem das kanülenseitige Ende des Spritzenzylinders (3) verschließenden Verschlussstopfen (15), der einen mittels einer Membran (19) verschlossenen Durchgangskanal (17) aufweist, eine an dem dem Verschlussstopfen (15) gegenüberliegenden Ende des Spritzenzylinders (3) angeordneten, eine Durchgangsoffnung (49) für die Kolbenstange (11) aufweisenden Fingerauflage (13) und mit einer Gewindeeinrichtung (25), die mit der Kolbenstange (11) und mit der Fingerauflage (13) zusammenwirkt, und die eine Gewindehülse (27) umfasst, die ein Innengewinde (59) aufweist, das mit dem Außengewinde (29) an der Kolbenstange (11) zusammenwirkt, **dadurch gekennzeichnet, dass** die Gewindehülse (27) mit der Fingerauflage (13) lösbar verbunden und gemeinsam mit der Kolbenstange (11) in den Innenraum (5) verlagerbar ist.

2. Spritze nach Anspruch 1, **dadurch gekennzeichnet, dass** die Gewindehülse (27) in den Grundkörper der Fingerauflage (13) eingepresst ist.

3. Spritze nach Anspruch 1 oder 2, **dadurch gekennzeichnet, dass** die Gewindehülse (27) formschlüssig mit der Fingerauflage (13) gekoppelt ist.

4. Spritze nach einem der vorhergehenden Ansprüche, **dadurch gekennzeichnet, dass** ein mit der Membran (19) in Eingriff bringbarer Dorn (23) vorgesehen ist.

5. Spritze nach einem der vorhergehenden Ansprüche, **dadurch gekennzeichnet, dass** die Gewindeeinrichtung (25) als separates Teil ausgebildet ist.

## Claims

1. Prefilled syringe for medical purposes, with a syringe cylinder (3) enclosing an interior (5), a syringe plunger (9) arranged therein which can be displaced by a plunger rod (11) having an outer thread (29), a closure stopper (15) which seals the cannula end of the syringe cylinder (3) and has a through channel (17) which is sealed by a membrane (19), a finger support (13) arranged on the opposite end of the syringe cylinder (3) to the closure stopper (15) and having a through opening (49) for the plunger rod (11), and a threaded device (25) which interacts with the plunger rod (11) and the finger support (13) and comprises a threaded sleeve (27) having an inner thread (59) which interacts with the outer thread (29) on the plunger rod (11), **characterized in that** the threaded sleeve (27) is detachably connected to the finger support (13) and can be displaced into the interior (5) together with the plunger rod (11).

2. Syringe according to claim 1, **characterized in that** the thread sleeve (27) is pressed into the base of the finger support (13).

3. Syringe according to claim 1 or 2, **characterized in that** the thread sleeve (27) is coupled with the finger support (13) in a positively locking manner.

4. Syringe according to any one of the preceding claims, **characterized in that** there is provided a pin (23) that can be brought into contact with the membrane (19).

5. Syringe according to any one of the preceding claims, **characterized in that** the thread system (25) is configured as a separate part.

## Revendications

1. Seringue préremplie à des fins médicales avec un cylindre de seringue (3) délimitant un espace intérieur (5), avec un piston de seringue (9) y étant disposé et déplaçable au moyen d'une tige de piston (11) présentant un filetage extérieur (29), avec un bouchon de fermeture (15) obturant l'extrémité du cylindre de seringue (3) du côté de la canule et laissant apparaître un canal traversant (17) fermé par une membrane (19), avec un appui-doigt (13) qui est percé d'une ouverture de passage (49) prévue pour la tige de piston (11) et qui est aménagé à l'extrémité du cylindre de seringue (3) à l'opposé du bouchon de fermeture (15) et avec un dispositif fileté (25) interagissant avec la tige de piston (11) et avec l'appui-doigt (13), et qui comprend une douille filetée (27), munie d'un filetage intérieur (59) s'engrenant sur le filetage extérieur (29) de la tige de piston (11), **caractérisée en ce que** la douille filetée (27) est solidaire de l'appui-doigt (13) d'une manière amovible et est déplaçable conjointement avec la tige de piston (11) dans l'espace intérieur (5).

2. Seringue selon la revendication 1, **caractérisée en ce que** la douille filetée (27) est enfoncée dans le corps de base de l'appui-doigt (13).

3. Seringue selon la revendication 1 ou 2, **caractérisée en ce que** la douille filetée (27) est accouplée à l'appui-doigt (13) par une liaison positive.

4. Seringue selon l'une des revendications précédentes, **caractérisée en ce qu'**un mandrin (23) est prévu pour être engagé avec la membrane (19).

5. Seringue selon l'une des revendications précédentes, **caractérisée en ce que** le dispositif fileté (25) est réalisé sous la forme d'une pièce séparée.
